(19) Europäisches Patentamt
European Patent Office
Office européen des brevets

(11) Publication number : **0 585 080 A2**

# (12) EUROPEAN PATENT APPLICATION

(21) Application number : **93306619.3**

(22) Date of filing : **20.08.93**

(51) Int. Cl.$^5$ : **C07D 323/06,** C07D 493/10, A61K 31/335

(30) Priority : **21.08.92 GB 9217765**

(43) Date of publication of application :
**02.03.94 Bulletin 94/09**

(84) Designated Contracting States :
**AT BE CH DE DK ES FR GB GR IE IT LI LU MC NL PT SE**

(71) Applicant : **OXACO S.A.**
**2, Rue Charles-Bonnet**
**CH-1206 Genève (CH)**

(72) Inventor : **Jefford, Charles William, c/o**
**Universite de Geneve**
**Section de Chimie, 30, quai Ernest-Ansermet**
**CH-1211 Geneve 4 (CH)**

(74) Representative : **Lamb, John Baxter**
**MARKS & CLERK 57-60 Lincoln's Inn Fields**
**London WC2A 3LS (GB)**

## (54) Oxygenated heterocyclic compounds, pharmaceutical compositions containing them.

(57) Compounds having antiparasitic properties are 1,2,4-trioxanes of the formula :

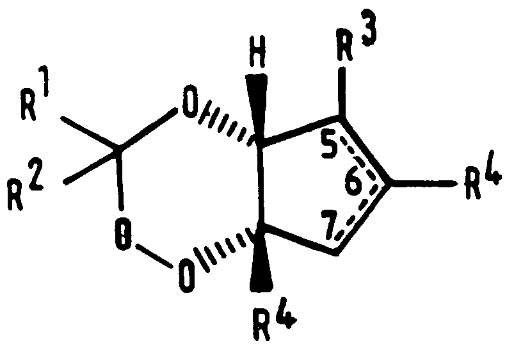

(VII)

in which :
$R^1$ and $R^2$ together with the adjacent carbon atom form a cycloalkyl group which may be interrupted by an oxygen atom ;
$R^3$ is a hydrogen atom or a hydroxyl gorup or etherified or esterified hydroxyl group, having the exo or the endo configuration, which, when an etherified, esterified or aminated group, may be further functionalized with a phosphate, phosphonate, carbamate or carboxylate group ;
$R^4$ is an aromatic substituent, optionally substituted with a halogen atom, an alkyl group, a trifluoromethyl group, an alkoxy-alkoxy group or an alkylamino group ; and the dotted lines between positions 5 and 6 and positions 6 and 7 indicate that there is present a 5,6- or 6,7- double bond.

EP 0 585 080 A2

This invention is concerned with improvements in and relating to new chemical compounds, the preparation thereof, and pharmaceutical compositions containing them.

It has now been found, in accordance with the present invention, that certain new 1,2,4-trioxane compounds, as hereinafter defined, possess anti-parasitic activity, as evidenced by in vitro and in vivo tests.

Accordingly, one embodiment of the invention provides, as new compounds, 1,2,4-trioxane derivatives of the formula:

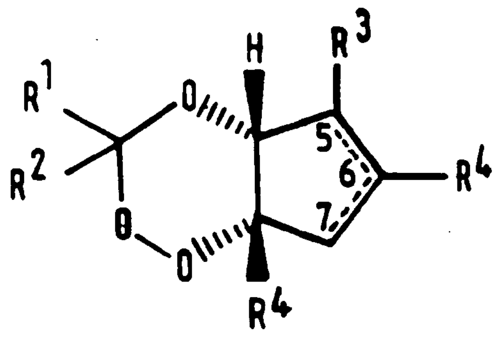

(VII)

in which:

$R^1$ and $R^2$ together with the adjacent carbon atom form a cycloalkyl group which may be interrupted by an oxygen atom;

$R^3$ is a hydrogen atom or a hydroxyl, etherified, esterified or aminated hydroxyl group, having the exo or the endo configuration, which, when etherified, esterified or aminated may be further functionalized with a phosphate, phosphonate, carbamate or carboxylate group;

$R^4$ is an aromatic substituent, usually a phenyl group, optionally substituted with a halogen atom, an alkyl group (e.g. a lower alkyl group), a trifluoromethyl group, an alkoxy-alkoxy group (e.g. a loweralkoxy-loweralkoxy group) or an alkylamino group (e.g. a lower alkylamino group); and

the dotted lines between positions 5 and 6 and positions 6 and 7 indicate that there is present a 5,6- or 6,7- double-bond.

When $R^3$ is an esterified hydroxyl group, it may, for example, be esterified with a lower aliphatic carboxylic acid; when an etherified hydroxyl group it may be etherified, for example, with a lower alkyl or aralkyl gorup; and when an aminated group, it may, for example, be animated with an amino-lower-alkyl or lower alkylamino group. In any event, the esterifying, etherifying or aminating group may be further functionalized, e.g. carry a phosphate, phosphonate, carbamate or carboxylate group.

Particular compounds in accordance with the invention are listed in the Table below.

| Compound | $R^1/R^2$ | $R^3$ *exo*, except where noted | $R^4$ | Double bond position |
|---|---|---|---|---|
| T13 | $-(CH_2)_4-$ | H | *p*-methoxymethoxy-phenyl | 5,6 |
| T16 | $-(CH_2)_4-$ | OH, *endo* | phenyl | 6,7 |
| T27 | $-(CH_2)_4-$ | OH | tolyl | 6,7 |
| T17 | $-(CH_2)_4-$ | OEt | phenyl | 6,7 |
| T18 | $-(CH_2)_4-$ | OBu | phenyl | 6,7 |
| T23 | $-(CH_2)_4-$ | $O-CCH_2CO_2Me$ | phenyl | 6,7 |
| T25 | $-(CH_2)_4-$ | $O-C(CH_2)_3CO_2H$ | phenyl | 6,7 |
| T52 | $-(CH_2)_2O(CH_2)_2-$ | OH | tolyl | 6,7 |
| T53 | $-(CH_2)_2O(CH_2)_2-$ | OOH | *p*-fluorophenyl | 6,7 |
| T54 | $-(CH_2)_2O(CH_2)_2-$ | OEt | phenyl | 6,7 |
| T55 | $-(CH_2)_2O(CH_2)_2-$ | $OCH_2O(CH_2)_2OMe$ | phenyl | 6,7 |
| T15 | $-(CH_2)_4-$ | OH | phenyl | 6,7 |
| T22 | $-(CH_2)_4-$ | $OCH_2C_6H_4CO_2H$-*p* | phenyl | 6,7 |
| T604 | $-(CH_2)_2O(CH_2)_2-$ | H | *p*-trifluoromethylphenyl | 5,6 |
| T623 | $-(CH_2)_4-$ | H | *p*-trifluoromethylphenyl | 5,6 |
| T630 | $-(CH_2)_2O(CH_2)_2-$ | H | *p*-methoxymethoxy-phenyl | 5,6 |
| T626 | $-(CH_2)_5-$ | OH | tolyl | 6,7 |
| T647 | $-(CH_2)_4-$ | $-O-C(CH_2)_3)CO_2H$ | phenyl | 6,7 |
| T13 | $-(CH_2)_4-$ | H | *p*-methoxymethoxy-phenyl | 5,6 |

The new compounds in accordance with the invention may conveniently be prepared by reaction of a compound of the formula:

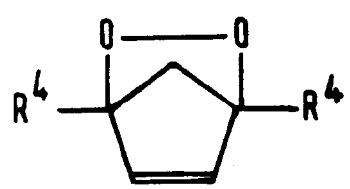

(IV)

(in which $R^4$ has the meaning defined above), with a cyclic ketone of the formula:

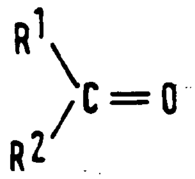

(V)

(in which $R^1$ and $R^2$ have the meanings defined above) to give a compound of the formula:

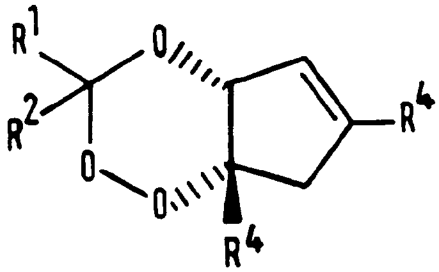

(VI)

followed, if desired, by conversion of the compound of formula (IV) (by peroxidation and reduction) to a hydroxyl compound of the formula:

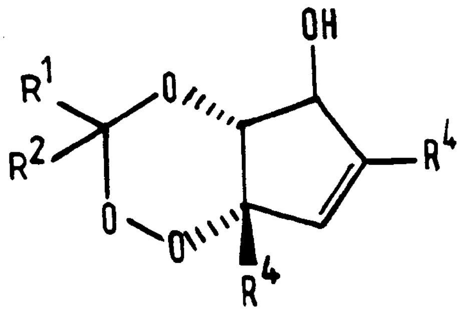

(VII)

and subsequent esterification, etherification or amination of the compound of formula (VII).

The starting compounds of formula (IV) may be prepared by a variety of routes. Thus, for example, they may be prepared from a 3-benzoylpropionic ester in accordance with scheme A in an improved version of the method of N.L. Drake and J.R. Andrews, Jr., (J.A.C.S., 1939 62 1326). Photo-oxygenation of the compound of formula (III) gives the desired compound of formula (IV).

Scheme A

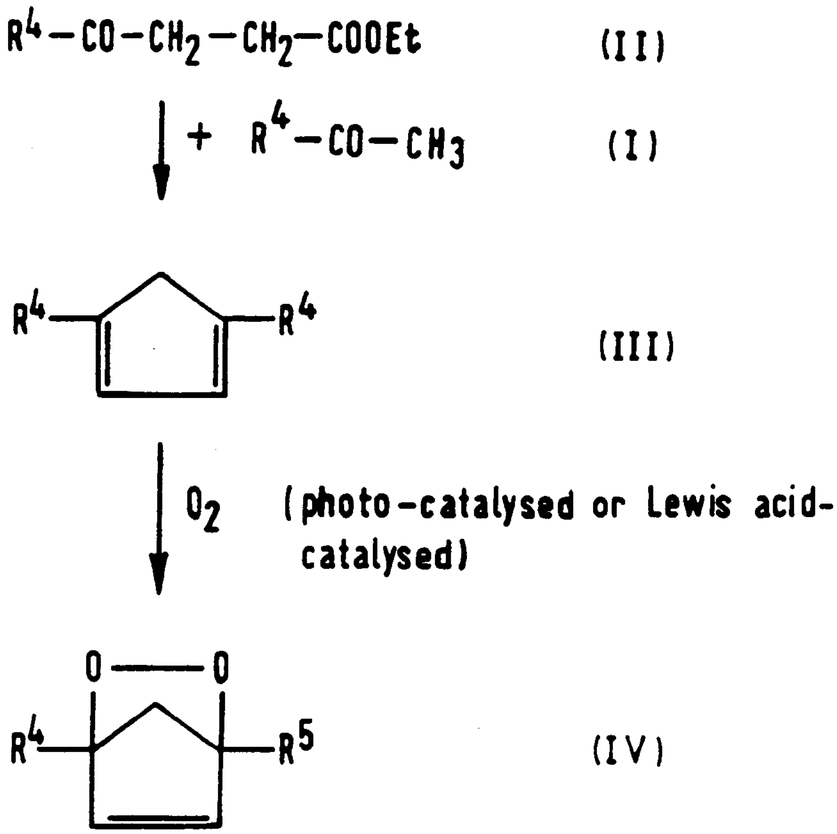


(II)

(I)

(III)

(IV)

Compounds of formula (II) may be prepared from an appropriately substituted benzaldehyde, in accordance with scheme B following the method of F.J. Mc.Evoy and J.D. Albright (J. Org. Chem., 1979, 44, 4597), or by Friedel-Crafts acylation of a suitably substituted benzene with succinic anhydride in accordance with Scheme C.

Scheme B

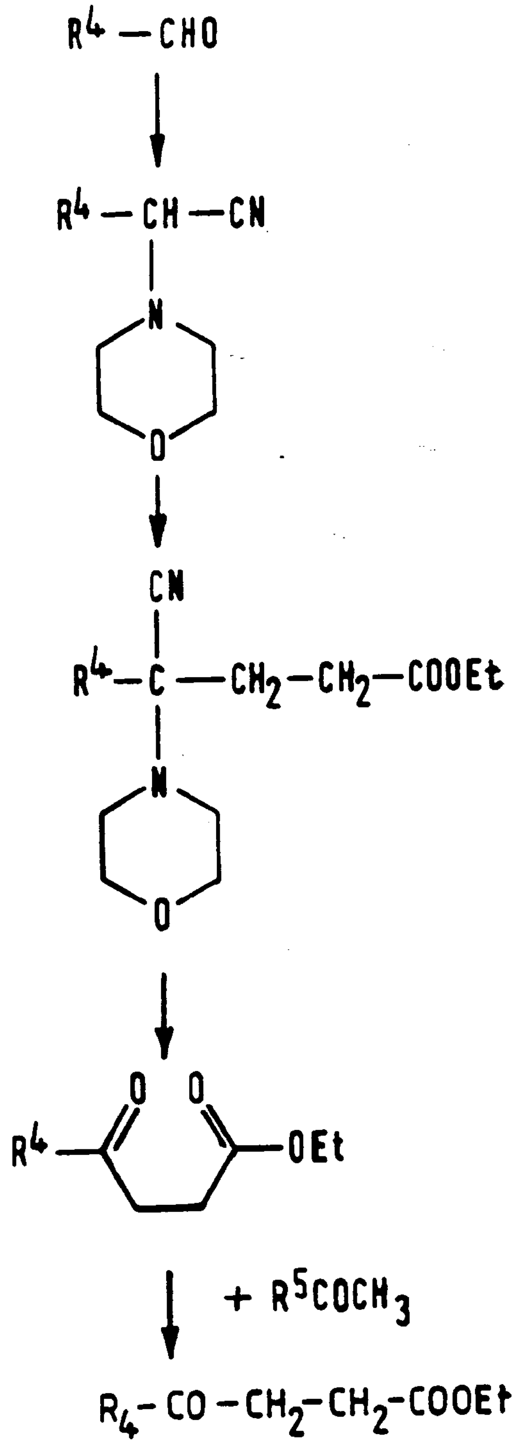


(II)

Scheme C

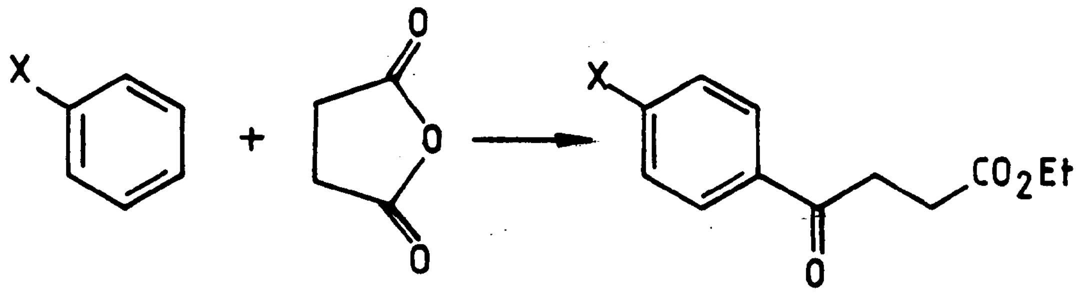

(II)

Compounds of formula (VII) may be prepared by dye-sensitized photo-oxygenation of compounds of formula (IV), followed by appropriate reduction in accordance with C.W. Jefford et al, Helv. Chim.Acta., 1986, 69, 941-948.

As noted above, compounds in accordance with the invention have anti-parasitic activity and thus, for example, may be used in the treatment of malaria or against pneumocystis carinii. Further, as a result of the anti-parasitic activity, the compounds may be used in the treatment of AIDS-related conditions.

The compounds in accordance with the invention have the particular advantage that, depending on the substituents, they may have lipophilic or hydrophilic properties which enable their activities to be modified as well as rendering their mode of application more versatile.

In view of the activity of the compounds, the invention also provides pharmaceutical compositions comprising the compounds of formula (VII) in association with a pharmaceutical carrier or diluent. The invention further provides the use of the compounds of formula (VII) in the preparation of pharmaceutical compositions for treatment of conditions as noted above.

Certain compounds of the invention were tested by the method of Peters *et al. (Ann. Trop. Med. Parasit.* **1975**, 69, 1550) in mice against *Plasmodium berghei,* N line, chloroquine-sensitive and *P. yoelii* ssp, NS line, chloroquine-resistant. Results are shown in Table 1 for the subcutaneous route and in Table 2 for the peroral route.

Table 1

| Compound | *exo* or *endo* | | *P. berghei* N | | *P. yoelii* NS | |
|---|---|---|---|---|---|---|
| | | | mg $kg^{-1}$ day x 4 sc | | | |
| | | | $ED_{50}$ | $ED_{90}$ | $ED_{50}$ | $ED_{90}$ |
| T13 | • | | 25.5 | 64.0 | 17.0 | 47.0 |
| T16 | | • | 6.1 | 10.1 | 6.7 | 11.2 |
| T27 | • | | 7.2 | 11.6 | 5.0 | 14.0 |
| T17 | • | | 8.5 | 18.5 | 8.8 | 24.2 |
| T18 | • | | 6.3 | 15.0 | 10.2 | 23.5 |
| T23 | • | | 13.0 | 26.0 | 23.5 | 60.0 |
| T25 | • | | 5.7 | 13.6 | 12.7 | 28.5 |
| T52 | • | | 4.2 | 17.0 | 7.0 | 15.0 |
| T53 | • | | 7.5 | 13.2 | 10.2 | 17.3 |
| T54 | | | | | 12.8 | 85.0 |
| T55 | • | | 3.8 | 7.5 | 4.5 | 11.5 |

Table 2

| Compound | *exo* or *endo* | | *P. berghei* N | | *P. yoelii* NS | |
|---|---|---|---|---|---|---|
| | | | mg $kg^{-1}$ day x 4 po | | | |
| | | | $ED_{50}$ | $ED_{90}$ | $ED_{50}$ | $ED_{90}$ |
| T15 | • | | 10.7 | 58.3 | | |
| T55 | | • | 13.4 | 23.0 | 5.3 | 25.5 |
| T22 | • | | 10.5 | 22.5 | | |

For other *in vivo* anti-malarial activities see W. Peters, B.L. Robinson, J.C. Rossier, D. Misra, C.W. Jefford, *Ann. Trop. Med. Parasit.* **1993**, *87*, 9.

Certain compounds of the invention were tested against *Plasmodium falciparum* clones *in vitro* according to the method of Desjardins *et al.* (R.E. Desjardins, C.J. Canfield, D.E. Haynes, J.D. Chulay, *Antimicrob. Agents. Chemother.* **1979**, *16*, 710; W.K. Milhous, N.F. Weatherly, J.H. Bowdre, R.E. Desjardins, *Antimicrob. Agents. Chemother.* **1985**, *27*, 525). The Indochina W2 clone is resistant to chloroquine, quinine, pyrimethamine and sulfadoxine but susceptible to mefloquine, whereas the Sierra Leone D6 clone is susceptible to chloroquine, quinine, pyrimethamine and sulfadoxine, but resistant to mefloquine. Results are shown in Table 3.

Table 3

| Compound | W2 clone | D6 clone |
|---|---|---|
| | IC-50 (ng/ml) | IC-50 (ng/ml) |
| T604 | 6.5 | 9.6 |
| T623 | 12.1 | 13.2 |
| T630 | 6.9 | 11.0 |
| T626 | 2.8 | 10.5 |
| T23 | 2.0 | 7.3 |
| T25 | 3.8 | 12.1 |
| T647 | 3.9 | 14.7 |
| T13 | 3.6 | 6.5 |

Certain compounds of the invention were tested *in vitro* against *Pneumocystis carinii* according to the method of M.S. Bartlett *et al.* (M.S. Bartlett, P.A. Verbanac, J.W. Smith, *J. Clin. Mi-crobiol.* **1979**, *10*, 796). Compounds T604, 630, 647 were strongly inhibited at 10 μg/ml.

In order that the invention may be well understood the following examples are given by way of illustration only.

EXAMPLES

General Procedure

**Preparation of 1,4-diphenylcyclopenta-1,3-diene (IIIa ($R^4$=Ph))**

Drake and Adams (see above) described the preparation of IIIa ($R^4$=Ph) by the condensation of acetophenone (Ia) ($R^4$=Ph) and ethyl 3-benzoylpropionate (IIa) ($R^4$=Ph) in the presence of sodium ethoxide. In their procedure sodium ethoxide and IIa are mixed together first of all. Then Ia is added and allowed to react on standing. Although the authors did not mention yields, their procedure in our hands always gave poor yields, usually 30% at best. It occurred to us that the mechanism of reaction must involve the initial formation of the enolate anion of Ia which then attacks IIa. Consequently, a better yield would be expected by first mixing Ia and sodium ethoxide and then adding IIa later. This expectation is confirmed by the new improved procedure.

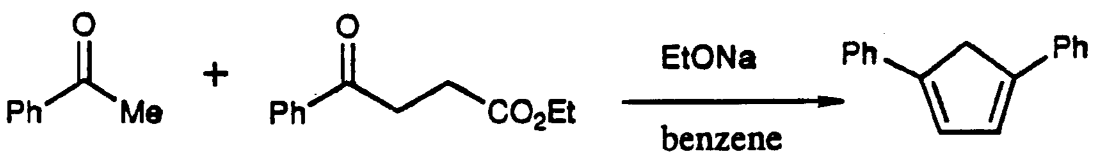


Ia IIa IIIa

**Improved procedure:**

To a three-necked flask was added dry ethanol (60 ml), followed by fresh sodium (5.8 g, 250 mmol) under an atmosphere of dry nitrogen with vigorous stirring. After addition, the reaction mixture was heated to ensure that the residual sodium had reacted completely. The ethanol was evaporated under reduced pressure, and dry benzene (120 ml) was added. The reaction mixture was cooled to 0°C with ice-water, and acetophenone (13.6 ml, 14 g, 117 mmol) in dry benzene (20 ml) was added slowly over 30 min., followed by the addition of ethyl 3-benzoylpropionate (24.24 g, 117 mmol) at r.t. The reaction mixture was then stirred at 40°C for 16 h and poured into 200 ml of ice-water. The aqueous layer was separated and the organic layer was washed with

brine several times. The combined aqueous layers were warmed at 70°C for 3 h to give IIIa as a yellowish precipitate. It was filtered off and washed successively with a little chilled ethanol and hexane. The aqueous filtrate was allowed to stand overnight, when another portion of IIIa precipitated. The product was pure enough for the next step (17.8 g, yield: 70.2%).

**Notes**:

1. TLC showed that IIIa was produced in the organic solvent before the reaction mixture was worked up.
2. It is possible to improve the yield by using a greater amount of acetophenone (1.2 eq.) at a lower temperature (-70°C).

Example 1

(a) α-[4-trifluoromethylphenyl]-4-morpholineacetonitrile

4-Trifluoromethylbenzaldehyde (2.0g, 11.5mmol) and morpholine (2.0g, 23mmol) were stirred in a water (30ml) /THF (20ml) mixture. To this was added p-toluenesulphonic acid (2.28g, 12mmol). After 10 minutes KCN (0.74g, 11.5mmol) in water (5ml) was added slowly and the solution heated to 70°C for 2.5 hours. The mixture was cooled, then poured onto 10% $K_2CO_3$ (150ml) and extracted with $CH_2Cl_2$. The organic phase was washed with 10% $NaHCO_3$ (3x150ml), dried and evaporated to give a yellow oil which was distilled to give α-[4-(trifluoromethyl)phenyl]-4-morpholineacetonitrile, 2.41g, 77.6% $^1$H-NMR (200 MHz, $CDCl_3$): 7.68 (s,4 x arom. H), 4.85 (s, CH(CN) ), 3.72 (m, morpholine 2 x $CH_2$), 2.58 (m, morpholine 2 x $CH_2$). $^{13}$C-NMR (50MHz, $CDCl_3$): 136.52 (s, Ar. C), 131.34 (q, J=33.4, <u>C</u>CF$_3$), 128.32 (s, Ar. $CH_2$), 125.8 (q, J=3.7, Ar.CH), 123.67 (q, J=271, $CF_3$), 114.45 (s, CN), 66.5 (s, $CH_2$), 61.99 (s, CH), 49.93 (s, $CH_2$). MS: 270 ($M^+$). Anal. calc. for $C_{13}H_{13}F_3N_2O$ (270): C 57.78, H 4.85, n 10.37; found: C 57.61, H 4.73, N 10.13.

(b) ethyl-γ-[4-tritluoromethyl(phenyl)]-γ-cyano-4-morpholinebutanoate

A solution of the morpholineacetonitrile (5.0g, 18.5mmol) in THF (50ml) was stirred at room temperature. To this was added 30% KOH in ethanol (1.4ml) and the solution then cooled to 0°C. Ethyl acrylate (3.6ml, 3.3g, 33 mmol) was added dropwise, the solution being allowed to rise to room temperature and stirred overnight. After evaporation of the solvent, petroleum ether was added. Filtration of the solid and evaporation of the filtrate gave, on standing, a solid, ethyl-γ-[4-trifluoromethyl(phenyl)]-γ-cyano-4-morpholinebutanoate 5.73g, 83.6%. $^1$H-NMR (60MHz, $CDCl_3$): 7.67 (m, 4 arom. H), 4.02 (q, J=7, $OCH_2CH_3$), 3.73 (m, 2 $CH_2$), 2.07.2.77 (m, 4 $CH_2$), 1.20 (t, J=7, $CH_3$).

(c) ethyl 3-(4'-trifluoromethylbenzoyl)propionate, II, ($R^4$=p-$CF_3C_6H_4$)

The morpholinebutanoate (5.6g, 15.1mmol) was taken up in 70% acetic acid (100cm$^3$) and heated at 75°C for 3 hours. The solution was cooled, extracted with chloroform and then washed with 10% $K_2CO_3$. Evaporation of this solvent gave a solid, ethyl 3-(4'-trifluoromethylbenzoyl)propionate. 3.7g, 89.2%. $^1$H-NMR (200MHz $CDCl_3$): 8.08 (d, J=8.5, 2 arom. H) 7.71 (d, J=8.5, 2 arom. H), 4.14 (q, J=7.1, $CH_2CH_3$), 3.30 (t, J=6.5, $CH_2$), 2.75 (t, J=6.5, $CH_2$), 1.24 (t, J=7.1, $CH_3$). $^{13}$C-NMR (50MHz $CDCl_3$): 197.24 (s, C), 172.64 (s, C), 139.21 (s, C), 134.77 (q, J=33.5, $CCF_3$), 128.36 (s, Ar. CH), 125.70 (q, J=3.6, Ar. CH), 123.25 (q, J=271, $CF_3$), 60.75 (s, $CH_2$), 33.63 (s, $CH_2$), 28.10 (s, $CH_2$), 14.14 (s, $CH_3$). MS: 274 ($M^+$). Anal. calc. for $C_{13}H_{13}F_3O_3$ (274): C 56.94, H 4.78; found C 56.92, H 4.72.

(d) 1,4-bis-(4-trifluoromethylphenyl)cyclopenta-1,3-diene, (III, $R^4$=p-$CF_3C_6H_4$)

Sodium ethoxide (1.84g, 27mmol) was stirred benzene (25ml) and 4-trifluoromethylphenyl methyl ketone (I, $R^4$ = $CF_3C_6H_4$) (2.43 g, 13.5 mmol) and the ester (II, $R^4$ = p-$CF_3C_6H_4$) (3.7g, 13.5 mmol) were successively added. The mixture was then heated at 40°C overnight. After cooling, the solution was extracted with water (200ml) and the aqueous phase heated at 70°C for 2 hours. A precipitate formed which was filtered off. This was purified by flash chromatography (hexane) to give 1,4-bis(4-trifluoromethylphenyl)cyclopentadiene 3.2 g, 67% M.p.: 174-175°C. IR (neat): 1610, 1325, 1165, 1120, 1070, 815. $^1$H-NMR (200MHz $CDCl_3$) 7.61 (m, 4 arom. H), 7.06 (s, 2H), 3.81 (s, $CH_2$). MS:354 ($M^+$). Anal. calc. for $C_{19}H_{12}F_6$ (354):C 64.41, H 3,41; found: C 64.32, H 3.36.

(e) 1,4-bis(4-trifluoromethylphenyl)-2-cyclopentene-1,4-endoperoxide (IV, $R^4$ = p-$CF_3C_6H_4$)

The diene (0.50g, 0.14mmol) was dissolved in $CCl_4$ (5ml) and photo-oxygenated for 3 hours at -5°C with tetraphenylporphorin as sensitiser. The solvent was evaporated to give a green solid, 40mg, 73.4% crude yield. It was used without purification for the following reactions.

(f) cis-3,4a,7,7a-tetrahydro-6,7a-bis-(4-(trifluoromethyl) phenyl)-3-spiro-(cyclopentane)-cyclopenta[1,2-e]-[1,2,4]trioxine T623

The endoperoxide (50mg, 0.13mmol) and cyclopentanone (0.125ml) were cooled to -78°C un $CH_2Cl_2$ (2ml). To this was added TMSOTf (5μl). After 3 hours $EtN_3$ (15μl) was added and stirred for a further 5

mins. The solution was poured onto ice water (20ml) and extracted with $CH_2Cl_2$. Evaporation of the solvent gave a red gum which was purified by preparative chromatography (EtOAc:Hexane/1.20) to give cis-3,4a,7,7a-tetrahydro-6,7a-bis-(4-(trifluoromethyl)phenyl)-3-spiro-(cyclopentane)-cyclopental[1,2-e]-[1,2,4]trioxine 50 mg, 82%. M.p.: 92°. IR (neat) 1620, 1325, 1000-1165. $^1$H-NMR (200MHz $CDCl_3$): 7.50-7.80 (m, 8 arom. H), 6.45 (m, 1H), 5.23 (m, 1H), 3.09 (m, 2H), 2.30-2.45 (m, 1H), 1.55-1.95 (m, 7H). $^{13}$C-NMR (100MHz $CDCl_3$): 145.58 (s, C), 141.23 (s, C), 137.91 (s, C), 130.45 (q, J=33, $\underline{C}CF_3$), 130.06 (q, J=33, $\underline{C}CF_3$), 127.39 (s, alkene CH), 126.43 (s, Ar. CH), 126.13 (s, Ar. CH), 125.61 (q, J=4, Ar. CH), 125.43 (q, J=4, Ar. CH), 124.08 (q, J=271, $CF_3$), 123.97 (q, J=271, $CF_3$), 113.57 (s, acetal C), 87.04 (s, C-O), 80.12 (s, HCO), 44.61 (s, $CH_2$), 36.12 (s, $CH_2$), 35.98 (s, $CH_2$), 24.40 (s, $CH_2$), 22.80 (s, $CH_2$). MS: 470 ($M^+$). Anal. calc. for $C_{24}H_{20}F_6O_3$ (470): C 61.28, H 4.29; found: C 61.08, H 4.32.

## EXAMPLE 2

cis-3,4a,7,7a-tetrahydro-6,7a-bis(4-(trifluoromethyl)phenyl)-3-spiro-(4-tetrahydropyran)-cyclopenta[1,2-e]-trioxine T 604

The endoperoxide (103mg, 0.27mmol) and tetrahydropyran-4-one (0.25 ml) were cooled to -78°C in $CH_2Cl_2$ (4ml). To this was added TMSOTf (12µl). After 3 hours $EtN_3$ (30µl) was added and stirred for a further 5 mins. The solution was poured onto ice water (40ml) and extracted with $CH_2Cl_2$. Evaporation of the solvent gave an oil which was titrated to give a colourless solid. This was purified by preparative chromatography (EtOAc: Hexane/1.4) followed by recrystallisation from ethanol to give a colourless solid, cis-3,4a,7,7a-tetrahydro-6,7a-bis(4-(trifluoromethyl)phenyl)-3-spiro-(4-tetrahydropyran)cyclopental(1,2-e]-[1,2,4]trioxine, 64 mg, 49%. M.p.: 170-171°. IR (nujol): 1620, 1340, 1110. $^1$H-NMR (400MHz$CDCl_3$) 7.51-7.69 (m, 8 arom. H), 6.44 (m, 1H), 5.19 (m, 1H), 3.57-3.79 (m, 4H), 3.10 (m, 2H), 2.05-2.24 (m, 2H), 1.67-1.75 (m, 2H). $^{13}$C-NMR (100MHz $CDCl_3$): 145.71 (s, C), 141.75 (s, C), 137.64 (s, C), 130.51 (q, J=32, $XCF_3$), 130.19 (q,J=32, $XCF_3$), 127.11 (s, alkene CH), 126.21 (s, Ar.CH), 126.16 (s, Ar, CH), 125.65 (q, J=4, Ar.CH), 125.52 (q, J=4, Ar.CH), 124.02 (q, J=270, $CF_3$), 123.93 (q, J=270, $CF_3$), 100.57 (s, acetal C), 87.77 (s, C-O), 79.34 (s, HCO), 64.89 (s, $CH_2$), 64.37 (s, $CH_2$), 44.84 (s, $CH_2$), 34.76 (s, $CH_2$), 34.12 (s, $CH_2$). MS: 467 ($M^+$-19). Anal. calc. for $C_{24}H_{20}F_6O_4$ (486): C59.26, H4.12; found: C59.20, H4.12.

The characteristics for other compounds so produced are set out below.

## T13

IR (neat): 2900, 1620, 1510, 1250, 1160, 1090, 1000, 890, 790.

$^1$H-NMR ($CDCl_3$):1.70-1.91 (m, 8H), 2.95 (d, J=16,1H), 3.23 (d, J=16,1H), 3.48 (s, 6H), 5.18 (s, 5H), 6.17 (d, J=3,1H), 7.4 (m, 8H).

$^{13}$C-NMR ($CDCl_3$): 23.0, 24.2, 35.7, 36.2, 43.7, 56.0, 80.0, 87.0, 94.0, 94.4, 113.0, 116.0, 116.2, 117.2, 120.7, 120.9, 122.7, 125.5, 127.1, 127.4, 128.8, 135.2, 156.8, 157.4.

MS: no $M^+$, 354, 338, 314, 165, 135, 45 (100).

Anal. calc. for $C_{26}H_{30}O_7$:C68.68, H 6.60;
found: C 68.28, H 6.37.

## T630

IR (neat): 3000, 1650, 1520, 1240, 1150, 1100, 1050, 1090, 890, 780.

$^1$H-NMR ($CDCl_3$):1.7-2.34 (m, 4H), 2.96 (d, J=16,1H), 3.24 (d, J=16,1H), 3.47 (s, 6H), 3.7 (m, 4H), 5.1 (s, 5H), 6.19 (d, J=3.1H), 7.4 (m, 8H).

$^{13}$C-NMR ($CDCl_3$):34.1, 34.9, 44.2, 56.0, 64.4, 64.9, 79.2, 87.6, 94.2, 94.3, 100.0, 116.0, 116.2, 122.7, 126.5, 126.8, 127.2, 127.3, 128.3, 128.4, 128.5, 135.15, 156.7, 157.4.

MS: 470 ($M^+$), 354, 338, 165, 135.45 (100).

Anal. calc. for $C_{26}H_{30}O_8$:C66.33, H 6.54.

Compounds T16, 16, 17, 18, 23, 25, 15, 22 and 647 were prepared by etherification or esterification of cis-±)-4a,7a-dihydro-exo-5-hydroxy-6,7a-diphenyl-spiro-(cyclopentane-3,3'-7H-cyclopenta-1,2,4-trioxin) with an appropriate organic chloride. Compounds T53-55 were prepared by etherification or esterification of the spirocyclic tetrahydropyran counterpart of the above trioxane.

Thus to an ice-cooled and stirred solution of NaH(80% in oil, 3 mmol) and dry THF(10 ml), was added dropwise the hydroxy trioxane (3 mmol, in 2 ml dry THF) while the temperature was maintained at 0°C. After 20 min. in the cooling bath, the chloride (4.5 mmol) was added and the reaction mixture was stirred at room temp. overnight.

The reaction mixture was poured into ice water and extracted with ether (3 X 25 ml). The combined ether extract was washed with water (2 X 10 ml), brine (10 ml) and dried ($Na_2SO_4$). The solvent was removed in vacuo and the residual reaction mixture was purified over a silica column using ethyl acetate:hexane (V/V 1/4) as eluent.

The compounds prepared had the characteristics noted below.

T17

IR (neat): 2900, 1450, 1350, 890, 780.

$^1$H-NMR ($CDCl_3$): 1.45 (t, J=7, 3H), 1.6-2.1 (m, 8h), 3.66 (m, 2H), 4.33 (d, J=3, 1H), 4.90 (m, 1H), 6.18 (s, 1H) 7.5 (m, 10H).

$^{13}$C-NMR ($CDCl_3$): 15.4, 23.5, 23.9, 35.2, 37.5, 65.5, 80.6, 86.7, 92.1, 113.9, 126.3, 126.8, 127.8, 127.9, 128.3, 128.4, 128.6, 133.6, 140.7, 146.0.

MS:378($M^+$), 278, 249, 233, 191, 105 (100), 77, 55.

Anal. calc. for $C_{24}H_{26}O_4$: C76.19, H 6.88; found: C76.05, H 6.90.

T22

The trioxane was prepared as a white solid by the general procedure and crystallized from 25% ethylacetate in hexane to give white crystals. mp: 161°C (86%).

IR ($CDCl_3$) : 3500-2800, 1700, 1440, 1280, 1120.

$^1$HNMR($CDCl_3$ : 1.7-2.2 (m, 8H), 4.50 (d, J=2, 1H), 4.75 & 4.83 (2xd), J=12, each for 1H), 5.1 (d, J=2, 1H), 6.36 (s, 1H), 7.3-8.01 (m, 14H).

$^{13}$CNMR($CDCl_3$): 23.70, 23.94, 35.11, 37.37, 71.25, 79.86, 86.82, 92.01, 113.82, 126.7, 126.8. 127.4, 128.3, 128.4, 128.5, 128.8, 130.2, 133.59, 140.40, 143.89, 145.89, 171.50.

Mass : no $M^+$, 384, 249, 221, 191, 105, 77.

Analysis calculated for $C_{30}H_{28}O_6$(484): C74.38, H 5.82; found: C74.13, H 5.90.

T25

The trioxane was prepared as a white solid by the general procedure and crystallized from CH2Cl2/hexane to give white crystals. mp: 122-123°C (70%).

IR ($CDCl_3$) : 3500-2800, 1760, 1740, 1440, 1320, 1140.

$^1$H NMR($CDCl_3$) : 1.6-2.26 (m, 14H), 4.38 (s, 1H), 6.47 (s, 2H), 7.41 (m, 10H).

$^{13}$C NMR ($CDCl_3$) : 19.59, 23.77, 24.13, 32.60, 33.15, 35.46, 37.01, 79.55, 80.41, 91.11, 114.22, 126.50, 126.61, 128.47, 128.55, 128.75, 129.10, 129.78, 132.80, 139.73, 145.04, 172.43, 177.92.

Mass : no $M^+$, 264, 191, 115, 86, 55.

Analysis calculated for $C_{27}H_{28}O_7$ (464): C 69.82, H 6.08; found: C 69.60, H 6.18.

TR54 :- IR (neat): 2900, 1650, 1520, 1190, 890, 780.

$^1$H-NMR ($CDCl_3$ ): 1.26 (t,J=7,3H), 1.84-2.04 (m,4H), 3.4 (s,3H), 3.78 (m,6H),4.42 (s,1H), 4.97 (m,1H), 6.18 (s,1H), 7.6 (m,10H).

$^{13}$C-NMR ($CDCl_3$): 15.5, 32.7, 35.6, 64.8, 64.9, 65.8, 78.6, 87.8, 91.2, 100.3, 126.3, 126.8, 127.9, 128.4, 128.5, 128.9, 133.7, 141.1, 144.9.

MS: no $M^+$, 294, 278, 233, 220, 105 (96), 77 (100).

Anal. calc. for $C_{24}H_{26}O_5$: C 73.09, H 6.59; found: C 72.68, H 6.50.

TR55 :- IR (neat): 2900; 1620, 1500, 1200, 880, 780.

$^1$H-NMR ($CDCl_3$): 1.9-2.1 (m,4H), 3.38 (s,3H), 3.42.3.85 (m,8H), 4.49 (s,1H), 4.92 (dd,J=7,2,2H), 5.28 (m,1H), 6.24 (s,1H), 7.50 (m,10H).

$^{13}$C-NMR ($CDCl_3$): 31.5, 34.5, 58.1, 63.8, 65.8, 66.8, 70.7, 77.7, 84.9, 92.2, 92.3, 94.7, 125.6, 125.8, 127.1, 127.4, 127.6, 127.8, 127.9, 132.4, 139.7, 144.3.

MS: no $M^+$, 338, 233, 105, 89, 77, 59 (100).

Anal. calc. for $C_{26}H_{30}O_7$.1/2 $CH_2Cl_2$: C 64.57, H 6.24; found: C 64.21, H 6.39.

## Claims

**1.** 1,2,4-Trioxane derivatives of the formula:

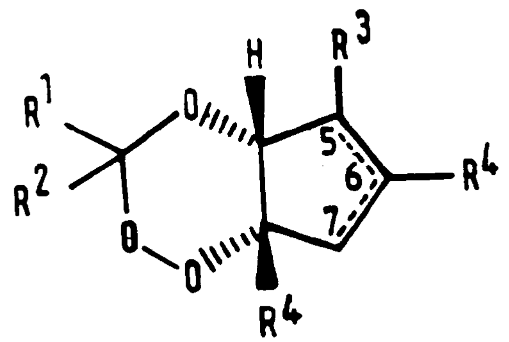

(VII)

in which:
$R^1$ and $R^2$ together with the adjacent carbon atom form a cycloalkyl group which may be interrupted by an oxygen atom;
$R^3$ is a hydrogen atom or a hydroxyl group or an etherified, esterified or aminated hydroxyl group, having the exo or the endo configuration, which, when an etherified hydroxyl group, may be etherified, esterified or aminated group may be further functionalized with a phosphate, phosphonate, carbamate or carboxylate group;
$R^4$ is an aromatic substituent, optionally substituted with a halogen atom, an alkyl group, a trifluoromethyl group, an alkoxy-alkoxy group or an alkylamino group; and the dotted lines between positions 5 and 6 and positions 6 and 7 indicate that there is present a 5,6- or 6,7- double bond.

**2.** Compounds as claimed in Claim 1 specifically as herein disclosed.

**3.** A pharmaceutical composition comprising a 1,2,4-trioxane compound as claimed in Claim 1 or Claim 2 together with a pharmaceutical carrier or diluent.

**4.** The use of a 1,2,4-trioxane compound as claimed in Claim 1 or claimed in the manufacture of an anti-parasitic pharmaceutical composition.